# EUROPEAN PATENT APPLICATION

(11) **EP 4 559 461 A1**
(43) Date of publication of application: **28.05.2025**
(21) Application number: 23383198.1
(22) Date of filing: 22.11.2023
(51) Int. Cl.: A61K 31/18, A61K 31/33, A61P 3/00, A61P 29/00

(54) **DUAL NLRP1 AND NLRP3 INHIBITORS FOR USE AS A MEDICAMENT**

(71) Applicant: Servicio Andaluz de Salud, 41071 Sevilla (ES); Universidad de Cádiz, 11002 Cádiz (ES); Universidad Pablo de Olavide, 41013 Sevilla (ES)
(72) Inventor: CORDERO MORALES, Mario, 41013 Sevilla (ES); BOTUBOL ARES, Jose Manuel, 11002 Cádiz (ES); DURÁN PEÑA, María Jesús, 11002 Cádiz (ES); GONZALEZ COLLADO, Isidro, 11002 Cádiz (ES); JIMÉNEZ TENORIO, Manuel, 11002 Cádiz (ES)
(74) Representative: Hoffmann Eitle

(57) **Abstract**

The present invention refers to the pharmaceutical field. Particularly, the present invention refers to dual NLRP1 and NLRP3 inhibitors with a N-arylmethyl arylalkylsulphonamide structure for use in medicine.

## Description

### FIELD OF THE INVENTION

The present invention refers to the pharmaceutical field. Particularly, the present invention refers to dual NLRP1 and NLRP3 inhibitors with a N-arylmethyl arylalkylsulphonamide structure for use in medicine. In a preferred embodiment, the dual NLRP1 and NLRP3 inhibitors are used in the treatment of diseases mediated by NLRP1 and/or NLRP3. These inhibitors have important applications in all those genetic, cardiovascular, metabolic, neurodegenerative, dermatological pathologies in which the activation of both inflammasomes has been shown to be involved.

### STATE OF THE ART

The NLRP3-inflammasome complex, one of the most studied major inflammatory pathways, has been associated with numerous diseases. In short, inflammasomes are cytosolic multiprotein complexes comprising an intracellular sensor, which is generally a NOD-like receptor (NLR), the protease precursor pro-caspase-1 and the adaptor protein ASC. Their activation leads to caspase-1 maturation and processing of its substrates, the immature pro-inflammatory cytokines pro-IL-1β and pro-IL-18, which results in the activation of a wide range of danger and stress signals.

In most of the diseases in which the NLRP3 complex has been described to be involved, the NLRP1 inflammasome complex is also active. NLRP1 and NLRP3 have been described to be involved in a wide variety of diseases, including:
- metabolic disorders, such as type 2 diabetes, atherosclerosis, obesity and gout.
- neurogenerative and neurological disorders such as Alzheimer's disease, Parkinson's disease, multiple sclerosis and muscular atrophic lateral sclerosis.
- pulmonary diseases such as asthma and COPD and idiopathic pulmonary fibrosis,
- liver diseases such as NASH syndrome, viral hepatitis and liver cirrhosis.
- pancreatic diseases such as acute and chronic pancreatitis, acute and renal diseases such as chronic kidney injury including kidney transplantation.
- intestinal diseases such as Crohn's disease and ulcerative colitis.
- skin diseases such as psoriasis, vitiligo or atopic dermatitis, musculoskeletal diseases such as scleroderma, vascular disorders such as giant cell arteritis.
- bone disorders such as osteoporosis, osteoarthritis and marble bone disease disorders, eye diseases such as glaucoma and luteal degeneration.
- diseases caused by viral infections such as HIV and AIDS, rheumatoid arthritis, systemic erythematosus, autoimmune thyroiditis.
- reproductive diseases such as ovarian aging, premature ovarian insufficiency, polycystic ovary syndrome, endometriosis, preterm labor, pre-eclampsia, and male subfertility.

In addition, they have been implicated in the development of complex diseases including malignant anemia, cancer and autoimmune diseases, as well as in aging and diseases of accelerated aging such as progeria.

Preliminary data from the inventors' group show that inhibition of NLRP3 also leads to compensatory activation of NLRP1 leading to increased inflammation, indicating that the inhibition of both inflammasomes at the same time is desirable. Additional scientific evidence shows a collaboration between NLRP3 and NLRP1, where prolonged treatment with NLRP3 inhibitors triggers a NLRP1 mediated compensatory effect.

In this context, there is an unmet medical need of developing dual pharmacologic inhibitors of NLRP1 and NLRP3 for the treatment of pathologies in which the activation of both inflammasomes are involved. The present invention is focused on solving this problem and dual NLRP1 and NLRP3 inhibitors are herein provided.

### DESCRIPTION OF THE INVENTION

### Brief description of the invention

As explained above, the present invention refers to dual NLRP1 and NLRP3 inhibitors with a N-arylmethyl arylalkylsulphonamide structure for use in medicine. In a preferred embodiment, the dual NLRP1 and NLRP3 inhibitors are used in the treatment of diseases mediated by NLRP1 and/or NLRP3.

The authors of the present invention have identified that various compounds with a N-arylmethyl arylalkylsulfonamide structure exhibit inhibition of NLRP1 and NLRP3 inflammasome complexes.

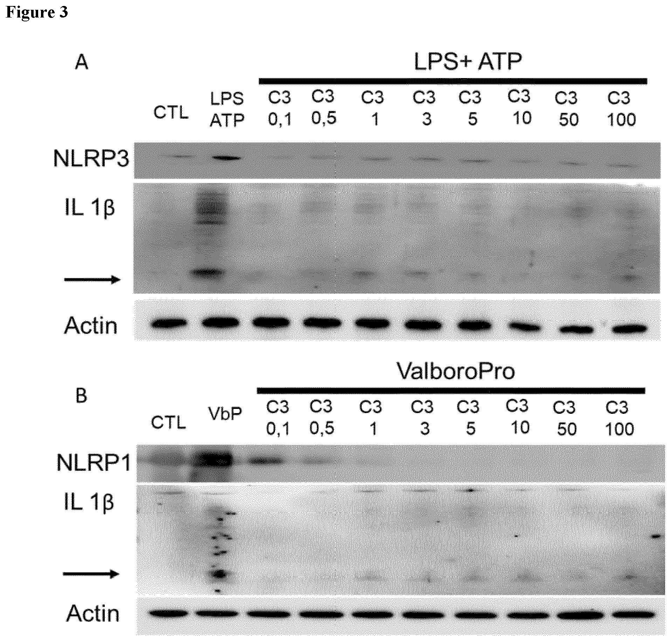

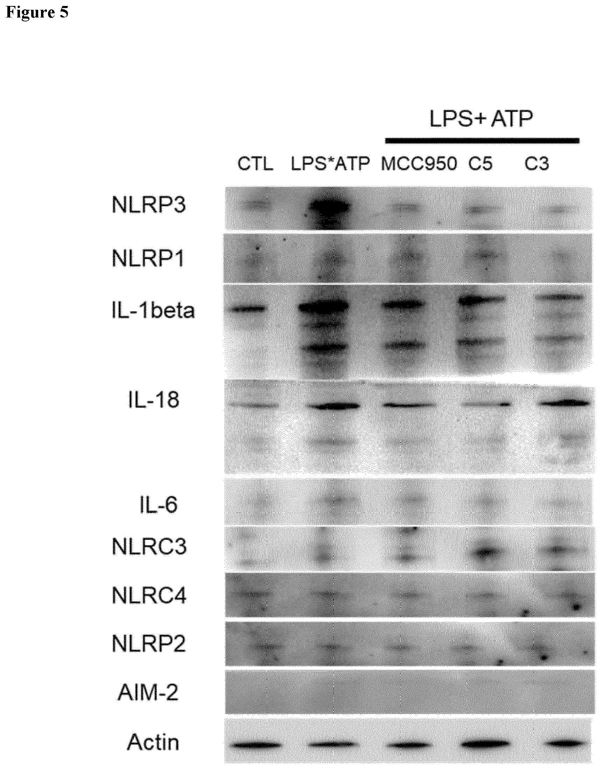

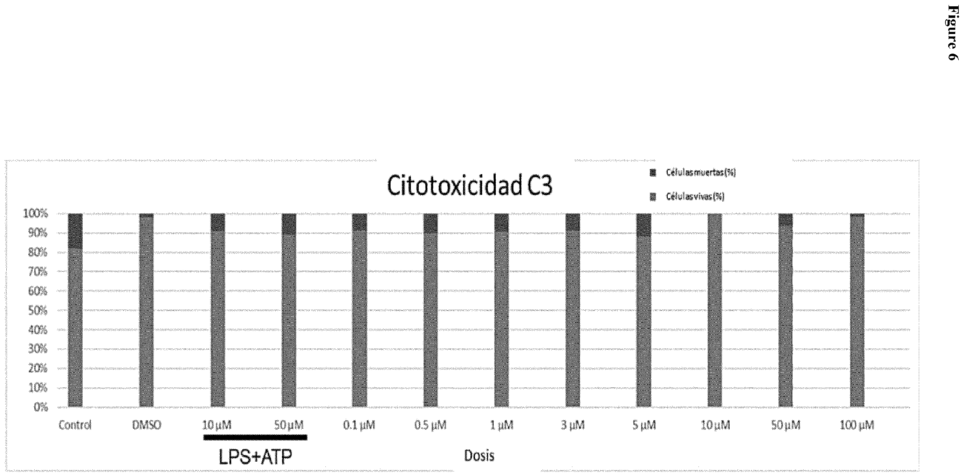

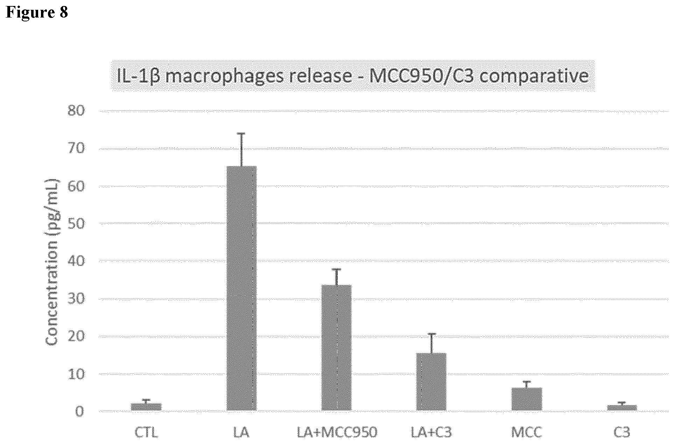

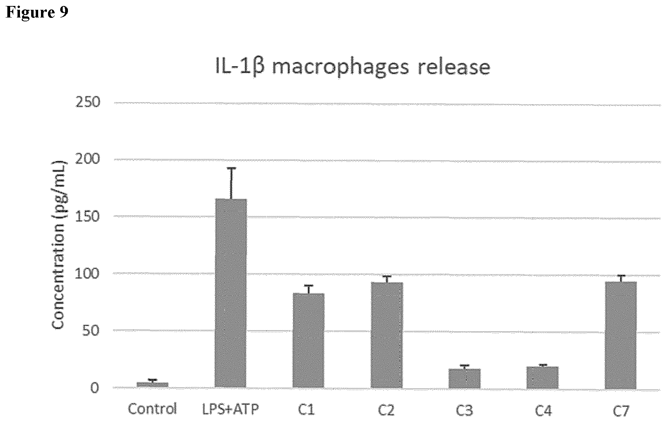

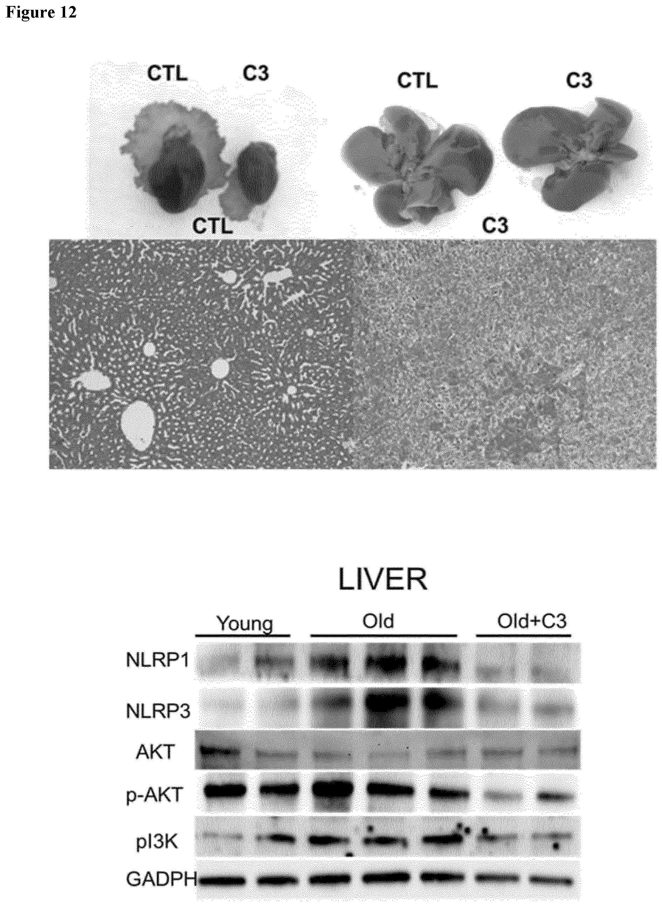

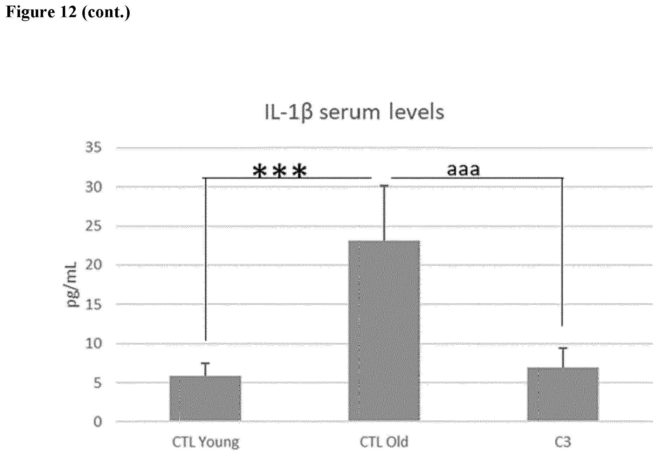

To evaluate the dual NLRP1 and NLRP3 inhibitory activity of the compounds, the inventors have performed *in vitro* (Example 2.1) and *in vivo* (Example 2.3) experiments. These experiments indicate that:
- treatment with compound C3 significantly reduces the expression of NLRP1 and NLRP3 in a dose-dependent manner in *in vitro* models of NLRP1 and NLRP3 activation (**Figure 3**), as well as in an *in vivo* ageing mouse model (**Figure 12**).
- treatment with compound C3 significantly reduces the expression of the inflammasome activation-associated cytokines IL-1β and/or IL-18 (**Figure 3**), as well as their release into the culture medium (**Figure 4B**). It also reduces serum IL-1 β levels in an *in vivo* ageing mouse model (**Figure 12**).
- compound C3 shows an increased inhibitory capacity *in vitro* compared to the existing NLRP3 selective inhibitor MCC950, as well as compared to a compound analogous to C3, N-benzylmethanesulfonamide (C5) (**Figure 5** and **Figure 8**).
- compound C3 does not have a toxic effect *in vitro* (**Figure 6**).
- treatment with compounds C1, C2, C4 and C7 reduces IL-1β cytokine release (**Figure 9**), indicating that the inhibitory activity of compound C3 is shared with other molecules derived from N-arylmethyl arylalkylsulfonamides.

Together, these results indicate that C1, C2, C3, C4 and C7 act as dual NLRP1 and NLRP3 inhibitors. Since these compounds share the N-arylmethyl arylalkylsulfonamides group, these results indicate the use of compounds sharing N-arylmethyl arylalkylsulfonamides group as dual inhibitors of NLRP1 and NLRP3 is plausible.

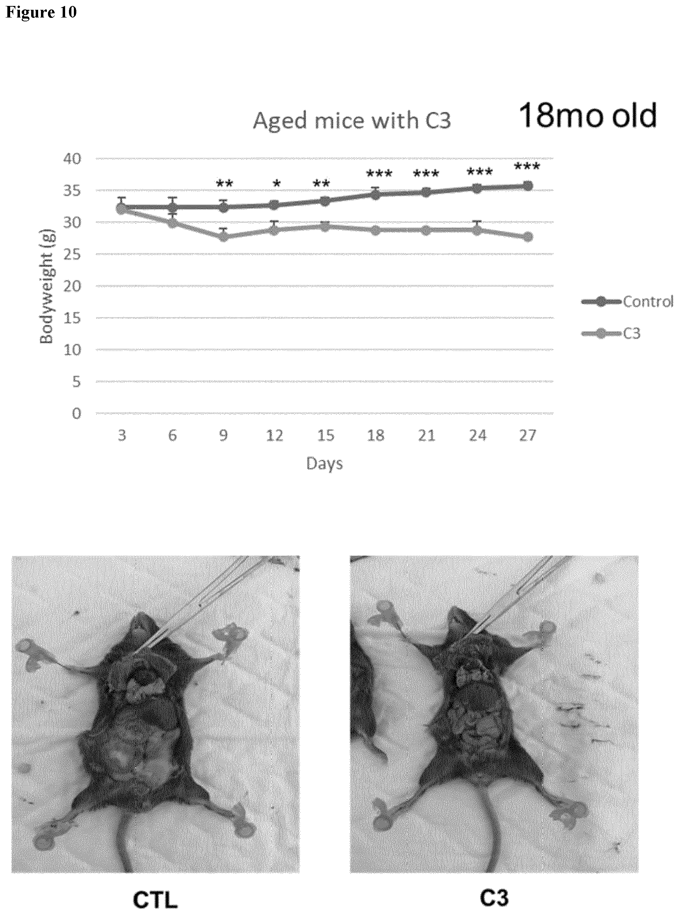

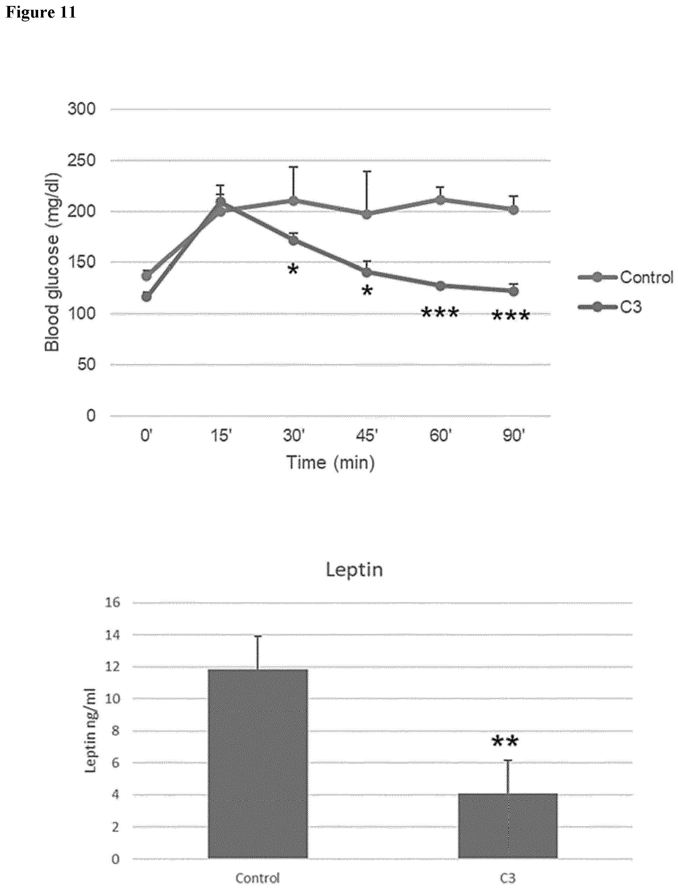

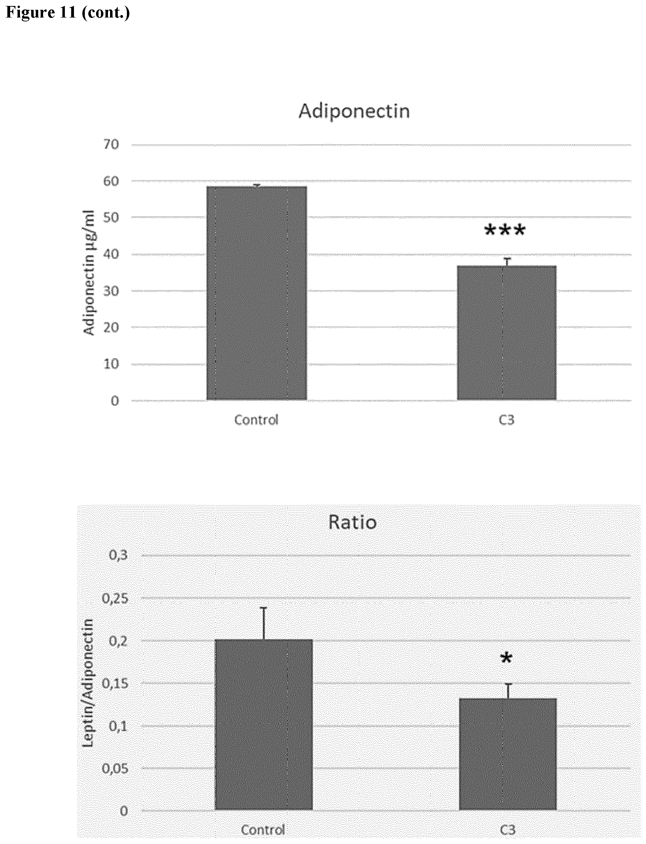

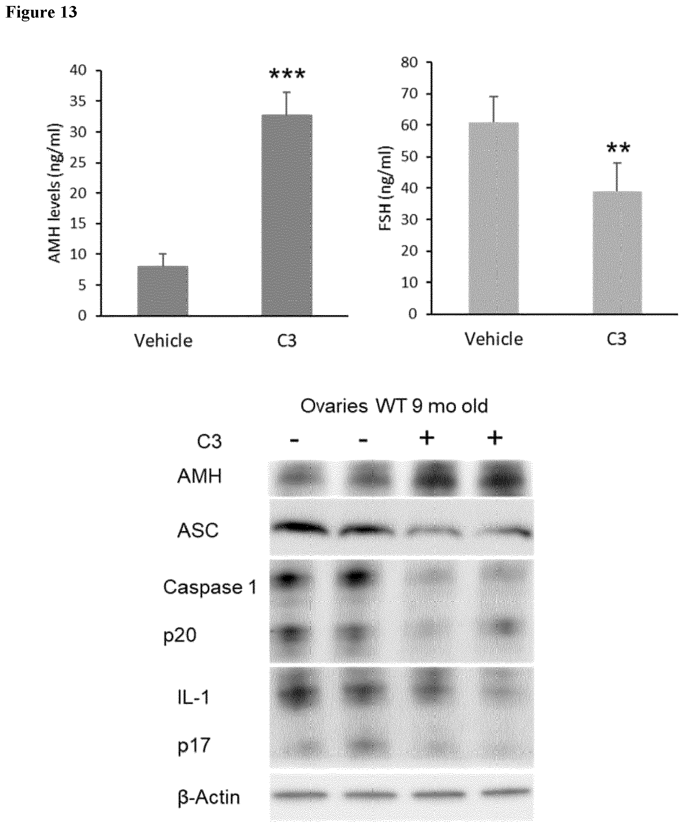

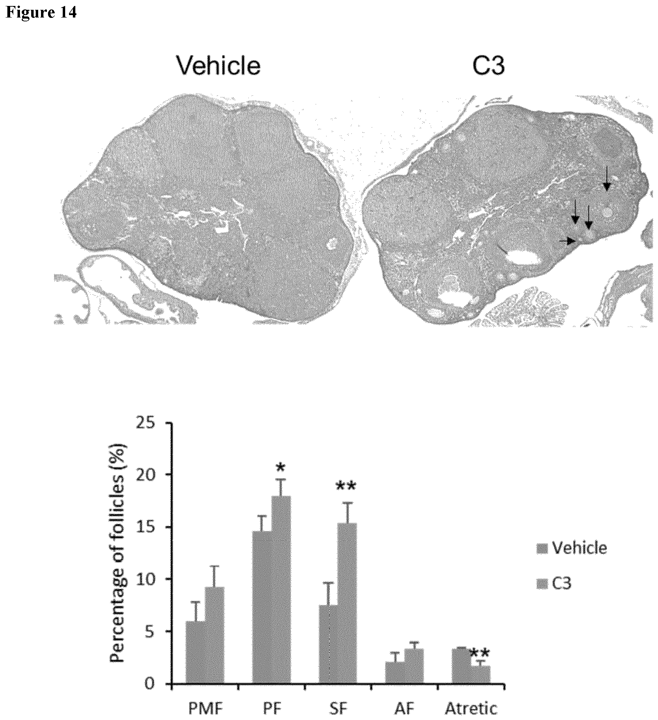

On the other hand, the inventors have also tested the ability of compound C3 to treat diseases mediated by NLRP1 and/or NLRP3. As a proof of concept, the inventors tested the effect of C3 treatment in mouse models of age-related metabolic dysfunction and ovarian ageing. These results indicate that:
- Treatment with C3 reduced body weight and abdominal fat (**Figure 10**), improves glucose and lipid metabolism in ageing mice (**Figure 11**), and reduced age-associated hepatic steatosis and fibrosis (**Figure 12**) in ageing mice, indicating that treatment with C3 improves age-related metabolic dysfunction.
- Treatment with C3 led to a significant increase in anti-müllerian hormone (AMH), a biochemical biomarker of ovarian reserve and AMH receptor protein levels, reduced the levels of follicle-stimulating hormone (FSH) (**Figure 13**). In addition, it also resulted in an increase in the number of follicles (**Figure 14**). These results indicate that treatment with C3 improves ovarian ageing.

So, the first embodiment of the invention refers to a dual NLRP1 and NLRP3 inhibitor characterized by *Formula I,* 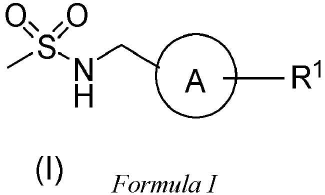 wherein:
a) ring A is selected from the group comprising C₆₋₁₀ aryls or heteroaryls having five or six members, and
b) R¹ is selected from the group comprising hydrogen, fluorine, chlorine, bromine, iodine, alkyl (C₁-C₄), hydroxyl or any of their pharmaceutically acceptable derivatives, or salts or derivates thereof,
for use as a medicament.

In a preferred embodiment, the dual NLRP1 and NLRP3 inhibitor characterized by *Formula I* is selected from: *Compound C1, Compound C2, Compound C3, Compound C4, Compound C5,* 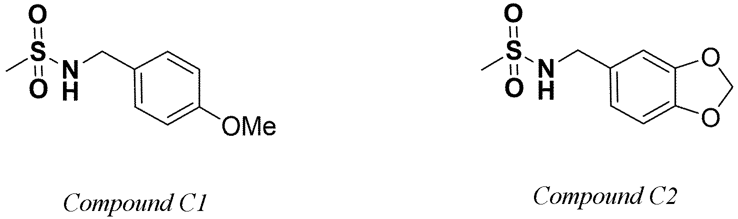 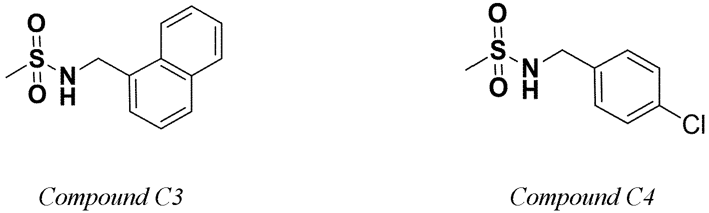 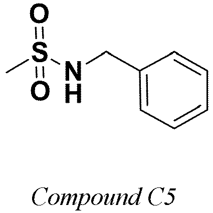 or a compound characterized by *Formula II,* 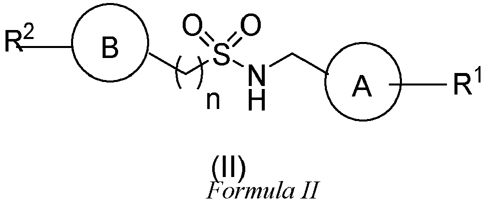 wherein:
a) rings A and B are selected from the group comprising C6-10 aryls or heteroaryls having five or six members,
b) n is 1 or 2, and
c) R¹ and R² are selected from the group comprising hydrogen, fluorine, chlorine, bromine, iodine, alkyl (C₁-C₄), hydroxyl or any of their pharmaceutically acceptable derivatives.

In a preferred embodiment, the dual NLRP1 and NLRP3 inhibitor characterized by *Formula II* is *Compound C7.*

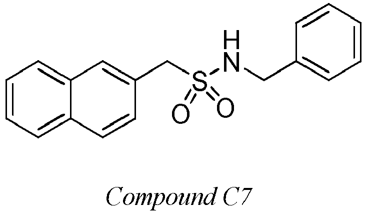

In a preferred embodiment, the dual NLRP1 and NLRP3 inhibitor is used in the treatment of diseases mediated by NLRP1 and/or NLRP3.

Kindly note that the diseases which are mediated by NLRP1 and/or NLRP3 pertain to the common general knowledge (see for instance the reference [Barnett KC, et al. A 360° view of the inflammasome: Mechanisms of activation, cell death, and diseases. Cell. 2023 May 25;186(11):2288-2312*].* So, the person skilled in the art would be able to establish the demarcation of the scope of the claim referring to diseases mediated by NLRP 1 and/or NLRP3 without undue burden.

In a preferred embodiment, the dual NLRP1 and NLRP3 inhibitor is used in the treatment of diseases mediated by NLRP1 and/or NLRP3 selected from: atherosclerosis, obesity, gout, type 1 diabetes, type 2 diabetes, insulitis, diabetic retinopathy, diabetic nephropathy, Alzheimer's disease, Parkinson's disease, multiple sclerosis, atrophic muscular lateral sclerosis, asthma, chronic obstructive pulmonary disease (COPD), idiopathic pulmonary fibrosis, non-alcoholic steatohepatitis (NASH), viral hepatitis, liver cirrhosis, acute pancreatitis, chronic pancreatitis, chronic kidney injury, Crohn's disease, ulcerative colitis, psoriasis, vitiligo, atopic dermatitis, scleroderma, giant cell arteritis, osteoporosis, osteoarthritis, marble bone disease disorders, glaucoma and luteal degeneration, eye diseases caused by viral infections, rheumatoid arthritis, systemic lupus erythematosus, autoimmune thyroiditis, malignant anemia, cancer and autoimmune diseases, progeria, side effects of chemotherapy, aging, infertility by ovarian aging, age-related metabolic dysfunction and ovarian aging.

In a preferred embodiment, the dual NLRP1 and NLRP3 inhibitor is used in the treatment of age-related metabolic dysfunction and/or ovarian aging.

Alternatively, the present invention also refers to a method for treating diseases mediated by NLRP1 and/or NLRP3, preferably age-related metabolic dysfunction and/or ovarian aging, more preferably atherosclerosis, obesity, gout, type 1 diabetes, type 2 diabetes, insulitis, diabetic retinopathy, diabetic nephropathy, Alzheimer's disease, Parkinson's disease, multiple sclerosis, atrophic muscular lateral sclerosis, asthma, chronic obstructive pulmonary disease (COPD), idiopathic pulmonary fibrosis, non-alcoholic steatohepatitis (NASH), viral hepatitis, liver cirrhosis, acute pancreatitis, chronic pancreatitis, chronic kidney injury, Crohn's disease, ulcerative colitis, psoriasis, vitiligo, atopic dermatitis, scleroderma, giant cell arteritis, osteoporosis, osteoarthritis, marble bone disease disorders, glaucoma and luteal degeneration, eye diseases caused by viral infections, rheumatoid arthritis, systemic lupus erythematosus, autoimmune thyroiditis, malignant anemia, cancer and autoimmune diseases, progeria, side effects of chemotherapy, aging, infertility by ovarian aging, age-related metabolic dysfunction and/or ovarian aging; which comprises administering a therapeutically effective dose or amount of the compounds of the invention or a pharmaceutical composition comprising thereof along with pharmaceutically acceptable excipient or carrier.

In the context of the present invention the following terms are defined:
- The term "comprising" means including, but it is not limited to, whatever follows the word "comprising". Thus, use of the term "comprising" indicates that the listed elements are required or mandatory, but that other elements are optional and may or may not be present.
- By "consisting of" means including, and it is limited to, whatever follows the phrase "consisting of'. Thus, the phrase "consisting of" indicates that the listed elements are required or mandatory, and that no other elements may be present.
- The term "derivatives" refers to any compound which, being administered to a recipient, is capable of providing (directly or indirectly) a compound described herein.
- The term "alkyl" refers, in the present invention, to saturated linear or branched hydrocarbon chains having from 1 to 4 carbon atoms (methyl, ethyl, n-propyl, i-propyl, n-butyl, tert-butyl, sec-butyl, or isobutyl).
- The term "aryl" refers, in the present invention, to aromatic carbocyclic systems containing 1 or 2 rings, where the rings may be fused. If the rings are fused, one of the rings must be fully unsaturated and the other ring may be fully saturated, partially unsaturated or fully unsaturated. This term includes, but is not only limited to, phenyl, naphthyl, indanyl or 1,2,3,4-tetrahydronaphthalenyl.
- The term "heteroaryl" refers, in the present invention, to aromatic carbocyclic systems containing 1, 2, 3 or 4 heteroatoms selected from a list of N, O and S and containing 1 or 2 rings, where the rings may be fused. This term includes, but it is not only limited to, furanyls, thienyls, oxazolyls, thiazolyls, imidazolyls, pyrazolyls, triazolyls.
- "Pharmaceutically acceptable excipient or carrier" refers to an excipient that may optionally be included in the compositions of the invention and that causes no significant adverse toxicological effects to the patient.
- By "therapeutically effective dose or amount" of a composition comprising the compounds of the invention is intended an amount that, when administered as described herein, brings about a positive therapeutic response in a subject having diseases mediated by NLRP1 and/or NLRP3. The exact amount required will vary from subject to subject, depending on the species, age, and general condition of the subject, the severity of the condition being treated, mode of administration, and the like. An appropriate "effective" amount in any individual case may be determined by one of ordinary skill in the art using routine experimentation, based upon the information provided herein.

### Description of the figures

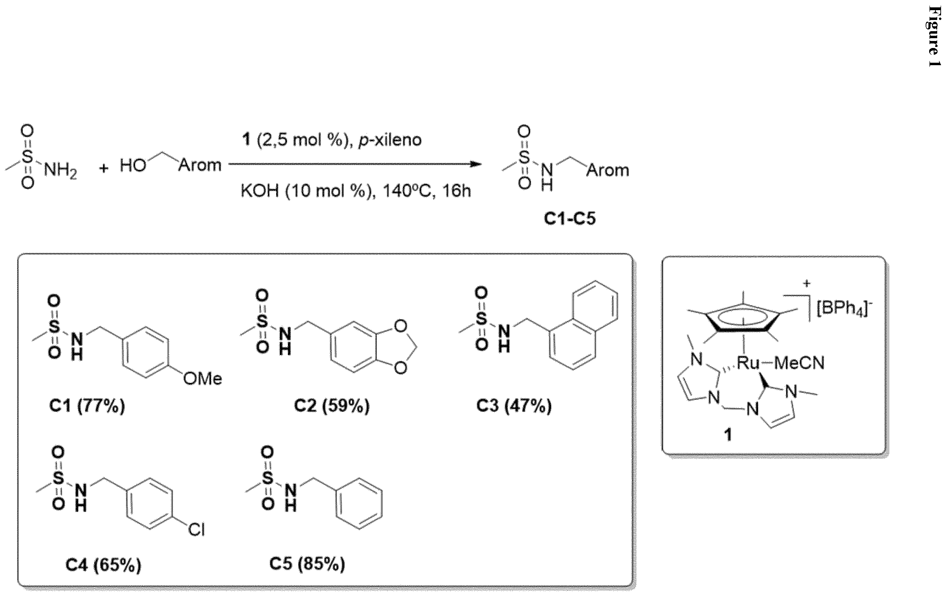

**Figure 1**. The figure illustrates the preparation of the compounds N-(4-methoxybenzyl) methanesulfonamide (**C1**), N-(4,5-methylenedioxybenzyl) methanesulfonamide (**C2**), N-(naphthalen-1-ylmethyl) methanesulfonamide (**C3**), N-(4-chlorobenzyl) methanesulfonamide (**C4**) and N-benzylmethanesulfonamide (**C5**) via N-alkylation of methanesulfonamide catalyzed by ruthenium complex 1. In addition, the structure of the catalyst is shown.

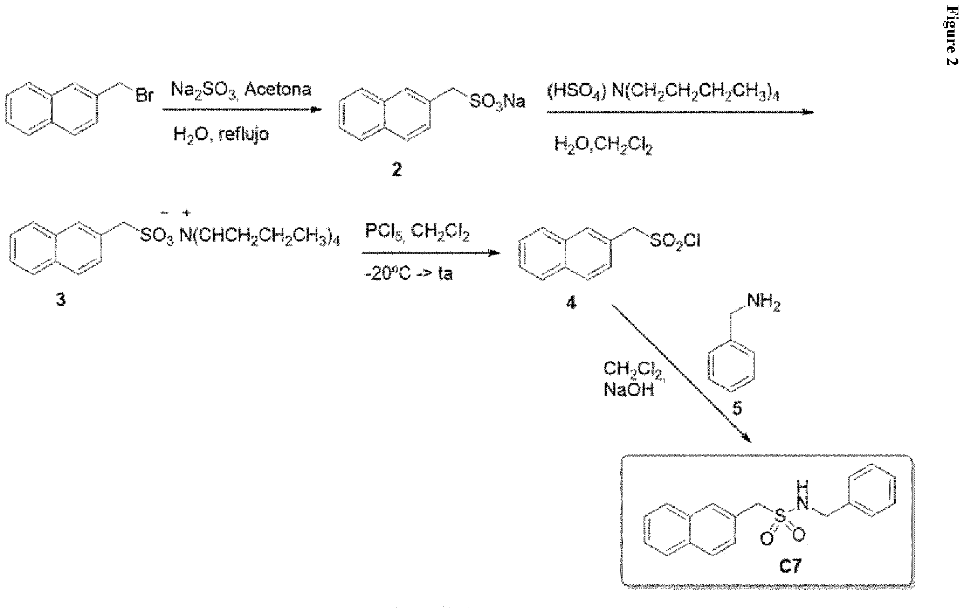

**Figure 2****.** The figure illustrates the synthesis sequence for the preparation of N-benzyl-1-(naphthalen-2-yl) methanesulfonamide (**C7**).
**Figure 3****.** The figure illustrates the effect of the compound **C3** on an *in vitro* model of NLRP3 and NLRP1 inflammasome activation following induction with (**A**) LPS+ATP, as a positive control model of NLRP3 activation, and (**B**) ValboroPro (VbP) as a positive control model of NLRP1 activation. It shows the effect of the compound **C3** on IL-1β, a cytokine that is activated by NLRP1 and NLRP3.

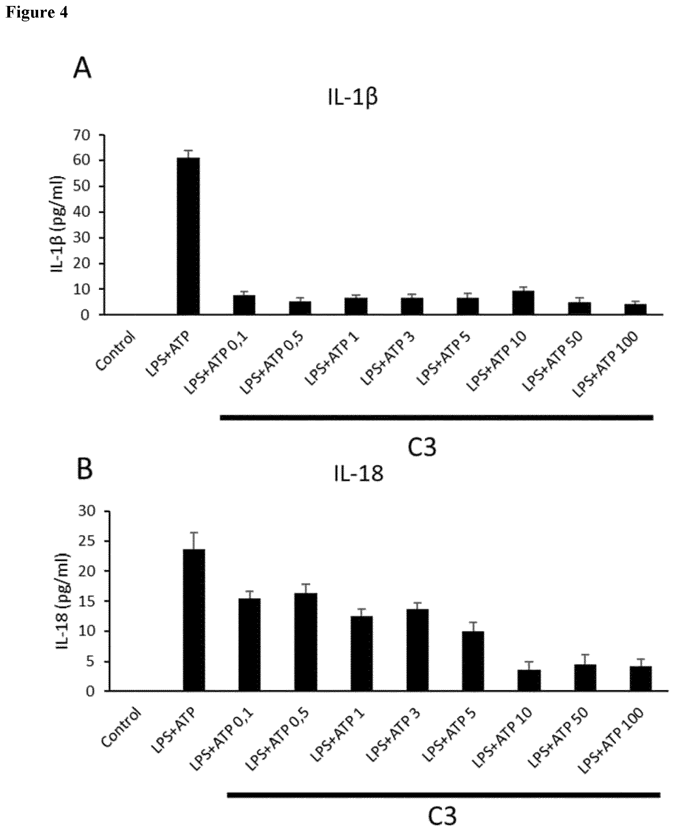

**Figure 4****.** The figure illustrates the levels of the cytokines IL-1β and IL-18 released into the medium as an effect of LPS+ATP mediated activation and the inhibitory potential of the compound.
**Figure 5****.** The figure illustrates a comparison of the effects of compound **C3,** C5 (another derivative compound), and the NLRP3 inhibitor MCC950 on the inhibition of different NLRP1 and NLRP3 inflammasome family proteins and other inflammatory proteins.
**Figure 6****.** The figure illustrates the toxicity of compound **C3** in an *in vitro* model.

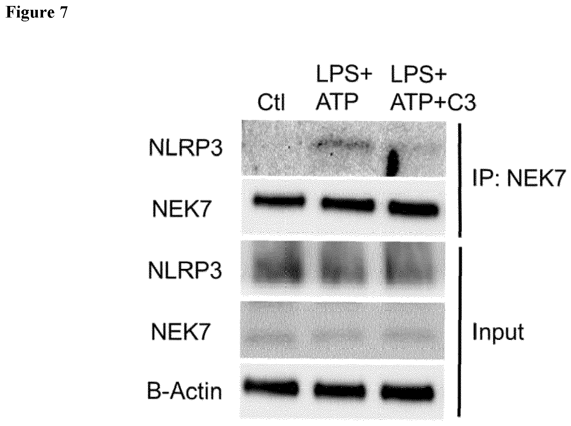

**Figure 7****.** The figure illustrates the results of NEK7 co-immunoprecipitation, where it can be observed that treatment with compound **C3** inhibits the binding of this protein to NLRP3.
**Figure 8****.** Effects of he figure illustrates the comparative of the effect of inhibitor compound C3 and MCC950 on IL-1β release.
**Figure 9****.** The figure illustrates the effect of compounds C1-C4 and C7 on IL-1β inhibition.
**Figure 10****.** The figure illustrates the show the effect of treatment with C3 on bodyweight in 18 months old mice.
**Figure 11****.** *In vivo* effects of treatment with C3 in glucose and lipid metabolism in mice.
**Figure 12****.** *In vivo* effects of treatment with C3 in hepatic steatosis and fibrosis, and in inflammasome activation.
**Figure 13****.** *In vivo* effects of treatment with C3 in the expression of biochemical biomarkers of ovarian research. AMH: anti-müllerian hormone, FSH: follicle-stimulating hormone.
**Figure 14****.** Histological staining that showing higher percentages of follicles and lower percentages of atretic follicles in C3-treated compared to vehicle-treated mice.

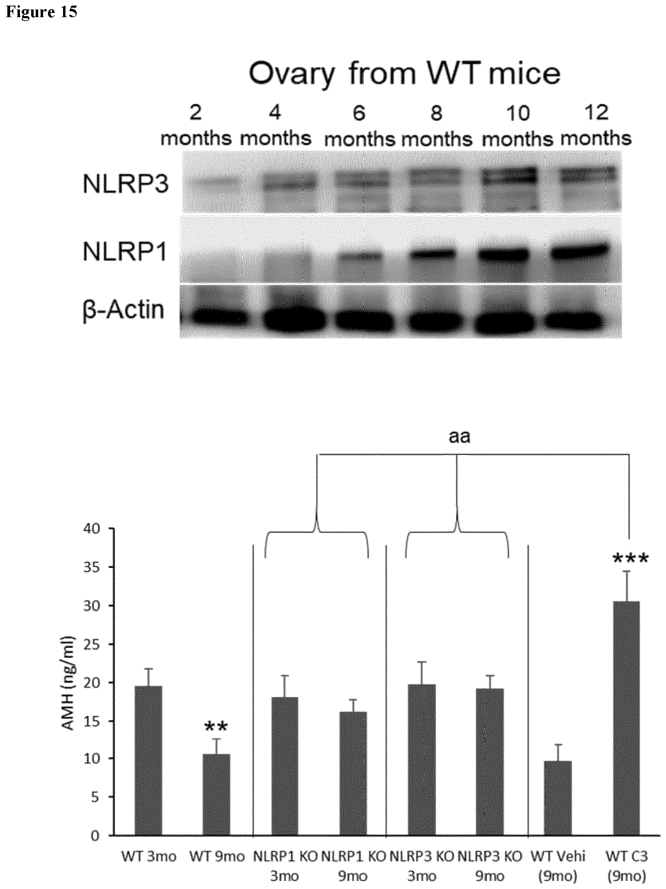

**Figure 15****.** Comparative study showing that treatment with C3 results in a more significant improvement of the ovarian reserve than NLRP 1 and NLRP3 genetic inactivation. AMH: anti-müllerian hormone.

### Detailed description of the invention

The present invention is illustrated by means of the Examples set below without the intention of limiting its scope of protection.

### Example 1. Material and Methods

### Example 1.1. Preparation of pentamethylcyclopentadienyl[bis(3-methylimidazol-2-ylidene)-methane](acetonitrile)ruthenium tetraphenylborate (II) (1)

The preparation of the ruthenium bis(NHC) catalyst was carried out by the procedure described in the literature [Botubol Ares J. M., Cordón-Ouahhabi, S., Moutaoukil, Z., Collado, I. G., Jiménez-Tenorio M., Puerta, M C., Valerga, P. Methylene-Linked Bis-NHC Half-sandwich ruthenium complexes: Binding of small molecules and catalysis toward ketone transfer hydrogenation, Organometallics, 2021, 40, 792-803*].*

### Example 1.2. Procedure for N-alkylation of methanesulfonamide. Preparation of compounds C1-C5 (Figure 1)

To a mixture of ruthenium catalyst 1 (39.3 mg; 0.05 mmol), KOH (28.1 mg; 0.50 mmol) and p-xylene (1 mL) under inert argon atmosphere at 140 °C was added the corresponding primary alcohol (1.00 mmol) and methanesulfonamide (95.1 mg; 1.00 mmol). After 24 h of stirring, the reaction crude was purified by column chromatography on silica gel eluting with increasing gradients of a hexane: ethyl acetate mixture to afford products C1-C5.

Physical and spectroscopic data for compound C1 (77% Rto) consistent with those described in the literature [Sanchez-Cantalejo, F., Priest, J. D., Davies, P. W. A Gold carbene manifold to prepare fused y-lactams by oxidative cyclisation of ynamides. Chem. Eur. J., 2018, 24, 17215-17219*].*

Physical and spectroscopic data for compound C2 (59% Rto) White solid (melting point 115°C); IR (KBr) vmax: 3279, 1607, 1504, 1431, 1311, 1252, 1102, 1037, 998, 934, 817 cm-1; NMR-1H (400 MHz, CDCl3) δH (ppm): 6.82 (m, 1H), 6.77 (m, 2H), 5.95 (s, 2H), 4.48 (s broad, 1H), 4.21 (d, J = 6.0 Hz, 2H), 2.87 (s, 3H); 13C NMR (100 MHz, CDCl3): δC (ppm): 148.1, 147.5, 130.4, 121.3, 108.4, 108.3, 103.4, 47.1, 41.2.

Physical and spectroscopic data for compound C3 (47% Rto) consistent with those described in the literature [Pedras, M. S. C., Jha, M. Toward the control of Leptosphaeria maculans: Design, syntheses, biological activity, and metabolism of potential detoxification inhibitors of the crucifer phytoalexin brassinin. Bioorg. Med. Chem. 2006, 14, 4958-4979*].*

Physical and spectroscopic data for compound C4 (65% Rto) consistent with those described in the literature [Xu, Q., Li, Q., Zhu, X, Chen, J. Green and scalable aldehyde-catalyzed transition metal-free dehydrative N-alkylation of amides and amines with alcohols. Adv. Synth. Cat. 2013, 355, 73-80*].*

Physical and spectroscopic data for compound C5 (85% Rto) consistent with those described in the literature [Moutaoukil, Z., Serrano-Díez, E., Collado I. G., Jiménez-Tenorio, M., Botubol Ares, J. M. N-Alkylation of organonitrogen compounds catalyzed by methylene-linked bis-NHC half-sandwich ruthenium complexes. Org. Biomol. Chem., 2022, 20, 831-839*].*

### Example 1.3. Obtaining the C7 compound (Figure 2)

### Example 1.3.1. Step 1: Preparation of sodium naphthalen-2-ylmethanesulfonate (2)

In a round bottom flask, 2-(bromomethyl)naphthalene (1.00 g; 4.53 mmol), acetone (8.4 mL), anhydrous sodium sulfite (628.2 mg; 4.98 mmol) and distilled water (2.0 mL) were added sequentially. The reaction was heated at 100 °C for 6 hours. The resulting crude was filtered under reduced pressure by washing first with acetone and then with distilled water. The crystalline solid obtained was left for 24 hours in the oven at 100 °C yielding 586.7 mg of compound 2 (55% Rto).

Physical and spectroscopic data for compound 2 consistent with those described in the literature (Walker, G., Rana, K. K. A novel preparation of 2-naphthyl and 5-benzo[B]thienyl methanesulfonyl chlorides. Synth. Comm., 2006, 33, 627-632).

### Example 1.3.2. Step 2: Preparation of tetrabutylammonium naphthalen-2-ylmethanesulfonate (3)

Sodium naphthalen-2-ylmethanesulfonate (1.53 g, 6.29 mmol), (CH₃CH₂CH₂CH₂CH₂)4NHSO₄ (2.01 g, 5.93 mmol), NaOH (0.26 g, 6.44 mmol), H₂O (15 mL) and dichloromethane (15 mL) were added sequentially in a round bottom flask and kept stirred at room temperature for 1h. The aqueous phase was extracted with dichloromethane (2 x 5 mL). The resulting organic phase was dried over anhydrous Na2SO4, filtered and the solvent was evaporated under reduced pressure yielding 3 (2.36 g, 79.8% Rto).

Physical and spectroscopic data for compound 3 consistent with those described in the literature *[*Al-Riyami, L., Pineda, M. A., Rzepecka, J., Huggan, J. K., Khalaf, A. I., Suckling, C. J., Scott, F. J., Rodgers, D. T, Harnett, M. M., Harnett, W. Designing anti-inflammatory drugs from parasitic worms: a synthetic small molecule analogue of the Acanthocheilonema viteae product ES-62 prevents development of collagen-induced arthritis. J. Med. Chem. 2013, 56, 9982-10002*].*

### Example 1.3.3. Step 3: Preparation of naphthalen-2-ylmethanesulfonyl chloride (4)

Tetrabutylammonium naphthalen-2-ylmethanesulfonate (3) (0.64 g, 1.39 mmol) was dissolved in dichloromethane (6 mL) and argon inert atmosphere in a round bottom flask and cooled with the aid of an inversion chiller to -20°C. Next, a solution of PCl5 (0.30 g, 1.44 mmol) in dichloromethane (6 mL) was added dropwise and the reaction was allowed to reach room temperature and stirred for an additional 30 minutes. The resulting crude was purified directly on silica gel, eluting with a mixture of hexane: AcOEt (50:50) to afford compound 4 (241.5 mg, 72.4% Rto).

Physical and spectroscopic data for compound 4 consistent with those described in literature *[*Al-Riyami, L., Pineda, M. A, Rzepecka, J., Huggan, J. K., Khalaf, A. I., Suckling, C. J., Scott, F. J., Rodgers, D. T, Harnett, M. M., Harnett, W. Designing anti-inflammatory drugs from parasitic worms: a synthetic small molecule analogue of the Acanthocheilonema viteae product ES-62 prevents development of collagen-induced arthritis. J. Med. Chem., 2013, 56, 9982-10002*].*

### Example 1.3.4. Step 4: Preparation of N-benzyl-1-(naphthalen-2-yl)methanesulfonamide (C7)

A solution benzylamine (5) (1.03 mmol) dissolved in dichloromethane (1 mL) and in an inert argon atmosphere was added at 0°C to a solution containing compound 4 (0.12 g, 0.51 mmol) in dichloromethane (1 mL). The reaction was allowed to reach room temperature and kept stirred for 3 hours. Then, the solvent was evaporated under reduced pressure and the resulting crude was dissolved in AcOEt (10 mL) and washed with 1 M HCl (2 x 5 mL). The resulting organic phase was dried over anhydrous Na2SO4, filtered and the solvent was evaporated under reduced pressure yielding a crude which is purified by column chromatography eluting with increasing gradients of hexane:AcOEt to give 59.1% Rto.

For biological assays, the THP-1 cell line (monocytes) was used and differentiated into macrophages using phorbol-12-myristate-13-acetate (PMA). They were then stimulated with 200 ng/mL ultrapure LPS for 4 hours, minus the negative control condition, to activate NLRP3 expression. At the same time, the necessary volume of the starting stock of each compound to be tested was added to reach the test concentration. After 4 h incubation at 37 °C, 5 mM ATP was added ATP 30 min and incubated for 30 min to activate NLRP3 assembly. For NLRP1 activation, Valboropro, a specific inducer of this protein was used at 1µM for 24 hours. Protein expression levels were studied by western blot as a proteomic study and inflammation levels by determination of cytokines released into the medium by immunoassay techniques (ELISA).

### Example 2. Results

### Example 2.1. In vitro characterization of the dual NLRP3 and NLRP1 inhibitory activity of C3

The inventors of the present invention evaluated the inhibitory effects of compound C3 in an *in vitro* model of NLRP3 induction (LPS + ATP), and in an *in vivo* model of NLRP1 induction (ValboroPro or Vbp). Compound C3 inhibited the expression of both NLRP3 and NLRP1 and reduced the expression of the inflammasome activation-associated protein IL-1β in a dose-dependent manner (**Figure 3**).

*In vitro* treatment with C3 also had a dose-dependent effect in IL-1β (**Figure 4A**) and IL-18 (**Figure 4B**) release into the culture medium. The estimated inhibitory capacity of the compound based on these determinations were IC50 = 5.38 nM (IL-1β) and IC50 = 4.45 µM (IL-18).

Compound C3 showed an increased inhibitory capacity compared to the existing NLRP3 selective inhibitor MCC950, as well as compared to a compound analogous to C3, N-benzylmethanesulfonamide (C5). This is reflected by the increased ability of C3 to inhibit the expression of NLRP1, NLRP3, IL-1β and IL-18 compared to MCC950 and C5 (**Figure 5** and **Figure 8**). Interestingly, treatment with Cr did not result in the modulation of other inflammasome proteins such as AIM-2, NLRP2, NLRC3 or NLRC4 (**Figure 5**).

In addition to this, *in vitro* experiments revealed that cell viability was above 85% regardless of the dose tested, indicating that treatment with C3 did not generate toxicity (**Figure 6**).

One of the possible mechanisms for NLRP3 inhibition is the inhibition of its binding to NEK7, since it has been shown that NEK7 interacts with NLRP3 to modulate NLRP3 inflammasome activation. Co-immunoprecipitation experiments revealed that the C3 inhibited the binding of NEK7 to NLRP3 (**Figure 7**), suggesting a possible mechanism through which C3 treatment could inhibit NLRP3 activity.

### Example 2.2. In vitro characterization of the dual NLRP3 and NLRP1 inhibitory activity of C1, C2, C4 and C7

*In vitro* treatment with compounds C1, C2, C4 and C7 also resulted in a reduction of IL-1β cytokine release (**Figure 9**), suggesting that the inhibitory activity of compound C3 is shared with other molecules derived from N-arylmethyl arylalkylsulfonamides.

### Example 2.3. In vivo characterization of the dual NLRP3 and NLRP1 inhibitory activity of the compounds

For a better characterization of the effect of C3, the inventors determined its effect in two *in vivo* models: ageing mice (to evaluate the effect in age-related metabolic dysfunction), and ovarian aging mice (to evaluate the effect in infertility because of ovarian aging).

### Example 2.3.1 Ageing mouse model

18-month-old C57BL/6J mice were treated with C3 (10 mg/Kg/day i.p.) or with a vehicle solution (saline) for over 4 weeks. C3-treated mice displayed significantly reduced body weight and abdominal fat compared to vehicle-treated mice (**Figure 10**).

C3 treatment also improved glucose metabolism, as indicated by the fact that glucose levels upon glucose challenges at the OGTT peak (>15 min) was significantly decreased in old mice treated with C3 compared to their control counterparts (**Figure 11**), indicating a higher rate of glucose clearance.

Leptin is an established regulator of body weight, leptin/adiponectin dysregulation has been associated with cardiovascular disease, metabolic syndrome, and non-alcoholic fatty liver disease. C3-treated old mice showed reduced serum levels of leptin, adiponectin and leptin/adiponectin (**Figure 11**).

Furthermore, age-associated hepatic steatosis and fibrosis were significantly reduced by C3 treatment as evidenced in microscopic liver sections. C3 treatment also led to a significant reduction in NLRP1 and NLRP3 protein expression, and serum IL1β levels (**Figure 12**).

### Example 2.3.2 Ovarian ageing mouse model

The second model used to evaluate the effect of C3 was related to ovarian aging. The reason behind this is that NLRP1 and NLRP3 is over-expressed during ovarian ageing (**Figure 15**).

For this, the inventors used 9-month-old adult mice to which C3 was administered for one month. C3 treatment led to a significant increase in antimüllerian hormone (AMH), a biochemical biomarker of ovarian reserve, a reduction of Follicle-stimulating hormone (FSH), and an increase of the AMH receptor. C3 also decreased protein levels of active Caspase 1 and active IL-1β (**Figure 13**).

Histological staining revealed a higher percentage of follicles and a lower percentage of atretic follicles in C3-treated compared to vehicle administrated mice (**Figure 14**).

Since NLRP1 and NLRP3 are over-expressed during ovarian aging (Figure 15), the inventors hypothesized that C3 could be improve the fertility in aged female mice by a dual inhibition of NLRP1 and NLRP3. In a comparative study using knock-out mice of NLRP1 and NLRP3, treatment with C3 showed a significantly higher improvement of the ovarian reserve by AMH hormone compared to NLRP1 KO and NLRP3 KO mice (**Figure 15**).

## Claims

1. Dual NLRP1 and NLRP3 inhibitor **characterized by** *Formula I,* 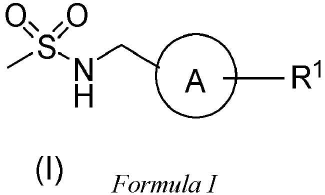 wherein:
c) ring A is selected from the group comprising C₆₋₁₀ aryls or heteroaryls having five or six members, and
d) R¹ is selected from the group comprising hydrogen, fluorine, chlorine, bromine, iodine, alkyl (C₁-C₄), hydroxyl or any of their pharmaceutically acceptable derivatives, or salts or derivates thereof,
for use as a medicament.

2. Dual NLRP1 and NLRP3 inhibitor for use, according to claim 1, wherein the dual NLRP1 and NLRP3 inhibitor **characterized by** *Formula I* is selected from: *Compound C1, Compound C2, Compound C3, Compound C4, Compound C5,* 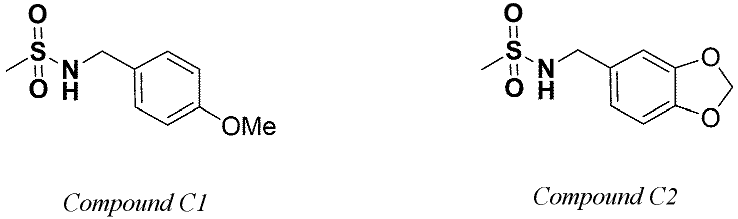 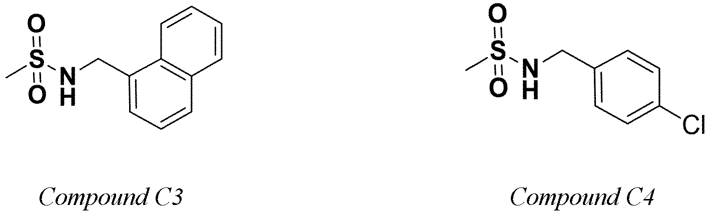 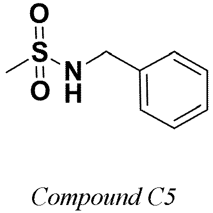 or a compound **characterized by** *Formula II,* 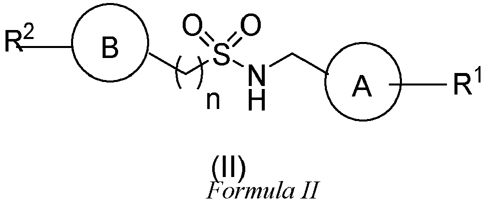 wherein:
d) rings A and B are selected from the group comprising C6-10 aryls or heteroaryls having five or six members,
e) n is 1 or 2, and
f) R¹ and R² are selected from the group comprising hydrogen, fluorine, chlorine, bromine, iodine, alkyl (C₁-C₄), hydroxyl or any of their pharmaceutically acceptable derivatives.

3. Dual NLRP1 and NLRP3 inhibitor for use, according to any of the previous claims, wherein the dual NLRP1 and NLRP3 inhibitor **characterized by** *Formula II* is *Compound C7.*

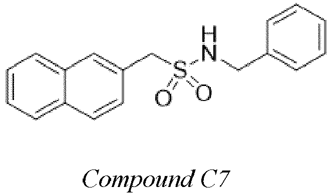

4. Dual NLRP1 and NLRP3 inhibitor for use, according to any of the previous claims, in the treatment of diseases mediated by NLRP1 and/or NLRP3.

5. Dual NLRP1 and NLRP3 inhibitor for use, according to claim 4, in the treatment of diseases mediated by NLRP1 and/or NLRP3 selected from: atherosclerosis, obesity, gout, type 1 diabetes, type 2 diabetes, insulitis, diabetic retinopathy, diabetic nephropathy, Alzheimer's disease, Parkinson's disease, multiple sclerosis, atrophic muscular lateral sclerosis, asthma, chronic obstructive pulmonary disease (COPD), idiopathic pulmonary fibrosis, non-alcoholic steatohepatitis (NASH), viral hepatitis, liver cirrhosis, acute pancreatitis, chronic pancreatitis, chronic kidney injury, Crohn's disease, ulcerative colitis, psoriasis, vitiligo, atopic dermatitis, scleroderma, giant cell arteritis, osteoporosis, osteoarthritis, marble bone disease disorders, glaucoma and luteal degeneration, eye diseases caused by viral infections, rheumatoid arthritis, systemic lupus erythematosus, autoimmune thyroiditis, malignant anemia, cancer and autoimmune diseases, progeria, side effects of chemotherapy, ageing, infertility by ovarian aging, age-related metabolic dysfunction and ovarian ageing.

6. Dual NLRP1 and NLRP3 inhibitor for use, according to claim 5, in the treatment of age-related metabolic dysfunction and ovarian ageing.

7. Dual NLRP1 and NLRP3 inhibitor for use, according to any of the previous claims, wherein dual NLRP1 and NLRP3 inhibitor is comprises in a pharmaceutical composition along with pharmaceutically acceptable excipients or carriers.
